# EUROPÄISCHE PATENTSCHRIFT

(11) **EP 0 548 620 B1**
(45) Veröffentlichungstag und Bekanntmachung des Hinweises auf die Patenterteilung: **19.04.1995**
(21) Anmeldenummer: 92120628.0
(22) Anmeldetag: 03.12.1992
(51) Int. Cl.: A61K 7/06, A61K 7/13

(54) **Verfahren zum oxidativen Färben von menschlichen Haaren und Mittel zur Durchführung dieses Verfahrens**
Process and composition for oxidative dyeing of human hair using catalase-free peroxidase
Composition et procédé de coloration oxidative des cheveux humains, utilisant une péroxidase dépourvue d'activité catalase

(30) Priorität: 20.12.1991 DE 4142278
(43) Veröffentlichungstag der Anmeldung: 30.06.1993
(73) Patentinhaber: GOLDWELL GmbH, D-64297 Darmstadt (DE)
(72) Erfinder: Dornbusch, Klaus, Dr., W-8771 Erlenbach (DE); Lorenz, Heribert, W-6101 Gross-Bieberau (DE); Olt, Andrea, W-6100 Darmstadt (DE); Tennigkeit, Jürgen, Dr., W-6104 Seeheim-Jugenheim (DE)

(56) Entgegenhaltungen:
- EP-A- 0 097 810
- EP-A- 0 310 675
- EP-A- 0 441 689
- DE-A- 2 155 390
- FR-A- 2 112 550
- US-A- 2 539 202
- US-A- 3 893 803
- US-A- 3 957 424
- Section Ch, Week 7819, 16. Juni 1978 Derwent Publications Ltd., London, GB;Class C, Page 015, AN 78-33901A

## Beschreibung

Die vorliegende Erfindung betrifft ein Verfahren zum oxidativen Färben von menschlichen Haaren und ein Mittel zur Durchführung dieses Verfahrens, das eine haarschonende Behandlung unter Vermeidung von Schädigungen gestattet.

Bekanntlich erfolgt die Oxidationsfärbung menschlicher Haare in zwei Stufen:
Die Vermischung einer Zusammensetzung, die mindestens ein Oxidationsfarbstoff-Vorprodukt sowie üblicherweise Kupplersubstanzen und gegebenenfalls Nuanceure enthält, mit einem Oxidationsmittel, wobei beide Zusammensetzungen bis zu dieser Vermischung getrennt gehalten wurden, und dem Aufbringen dieser gebrauchsfertig hergestellten Mischung auf menschliches Haar. Dabei kommen in der Regel Oxidationsmittelkonzentrationen von etwa 2,5 bis 5 Gewichtsprozent zum Einsatz, wobei vorwiegend Wasserstoffperoxid eingesetzt wird. Die Einwirkung derart hoher Oxidationsmittelkonzentrationen kann, insbesondere auch bei zusätzlich zuvor in alkalischem Medium dauergewelltem oder gebleichtem Haar, eine Haarschädigung hervorrufen.
Die Erfindung geht daher von der Aufgabenstellung aus, ein Mittel und ein Verfahren zum Färben von menschlichen Haaren zur Verfügung zu stellen, das eine schonende Haarbehandlung gewährleistet.

Die Lösung dieser Aufgabe besteht darin, eine Oxidationsmittelzusammensetzung anzuwenden, die Peroxide, insbesondere Wasserstoffperoxid, in einer wesentlich geringeren als der üblichen Konzentration, nämlich 0,01 bis 1 Gewichtsprozent, vorzugsweise 0,02 bis 0,2 Gewichtsprozent, insbesondere 0,05 bis 0,1 Gewichtsprozent, auf die Gesamtzusammensetzung und auf Wasserstoffperoxid berechnet, und zusätzlich eine katalasefreie Peroxidase enthält, d.h., deren Katalase-Gehalt weniger als 0,7 % der Gesamt-Peroxidase-Aktivität beträgt.

Durch die erfindungsgemäß erforderliche Abwesenheit der Katalase-Aktivität ist die Peroxidase bei der Anwendung geeignet, in geringen Mengen die Oxidation des Farbvorstoffproduktes durch geringe Mengen Peroxid zu katalysieren.
Durch die Verwendung des Peroxidase-Enzyms kann nicht nur die Menge des erforderlichen Oxidationsmittels drastisch reduziert werden; durch die dabei überraschenderweise mögliche Verkürzung des Färbevorgangs auf dem Haar von üblicherweise 30 auf 15 bis 20 Minuten wird eine weitere Haarschonung erzielt.

Um die Wirkung des erfindungsgemäßen Mittels noch zu verbessern, ist es zweckmäßig, den das Enzym enthaltenden Zusammensetzungen noch ein Benetzungsmittel zuzusetzen, um die Aufnahmefähigkeit der Haare zu verbessern.
Ein geeignetes Benetzungsmittel ist dabei ein nichtionisches Tensid, das in einer Menge von etwa 0,1 bis etwa 4,0 Gewichtsprozent zugesetzt werden kann.

Ein bevorzugtes nichtionisches Tensid ist insbesondere ein Ethylenoxid/Propylenoxid-Copolykondensat der allgemeinen Formel

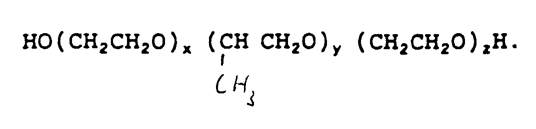

Ein besonders geeignetes Copolykondensat der obengenannten Formel ist ein solches, wo die Durchschnittswerte für x, y und z jeweils bei 98,67 und 98 liegen.
Ein derartiges Produkt ist unter der Bezeichnung "Poloxamer 407" bekannt und unter dem Handelsnamen "Genapol^{R} PF70" oder "Pluronic F127" erhältlich.
Geeignet sind jedoch auch andere "Poloxamer"-Typen der obengenannten allgemeinen Formel, wo die Mittelwerte für x und z zwischen 2 und 128 und diejenigen für y zwischen 16 und 67 liegen.

Geeignete Poloxamere sind im CTFA International Cosmetic Ingredient Dictionary, Fourth Edition (1991), S.447 bis 451, beschrieben.
Wie bereits ausgeführt, werden diese Tenside vorzugsweise in einer Konzentration zwischen 0,1 und 4 Gewichtsprozent, insbesondere 0,1 bis 2,5, ganz bevorzugt 0,5 bis 1,5 %, eingesetzt.

Weitere geeignete Tenside sind Alkylglycoside der allgemeinen Formel RO(OR¹)ₜZₓ, worin R eine Alkylgruppe mit 8 bis 18 Kohlenstoffatomen, R¹ einen Ethylen- oder Propylenrest, Z ein reduzierendes Saccharid mit 5 bis 6 Kohlenstoffatomen, t null bis 10 und x eins bis 5 bedeuten.
Auch diese Tenside sind geeignet, eine genügende Benetzbarkeit des Haares zu erreichen, ohne die Stabilität der Peroxidase zu beeinträchtigen.
Auch C₁₀-C₁₈-Alkylpolyglycolether, beispielsweise mit 5 bis 25 Ethylenoxid-Einheiten/Molekül, können, gegebenenfalls im Gemisch mit anderen Tensiden, eingesetzt werden. Solche Produkte sind beispielsweise unter dem Handelsnamen "Genapol^{R}" bekannt.

Es hat sich als vorteilhaft erwiesen, der Enzym- und/oder der Oxidationsmittel-Zusammensetzung noch ein Verdickungsmittel zuzusetzen, vorzugsweise in einer Menge von etwa 0,25 bis etwa 2,5, insbesondere etwa 0,5 bis etwa 2,0 Gewichtsprozent der jeweiligen Zusammensetzung.
Geeignete Verdickungsmittel sind insbesondere die bekannten Cellulosederivate wie Hydroxyethylcellulose, Agar-Agar, etc. Der Zusatz dieser Verdickungsmittel verbessert die Intensität der Ausfärbung.

Die erfindungsgemäß zum Einsatz gelangende Peroxidase kann pflanzlichen Ursprungs oder aus Bakterien gewonnen worden sein. Maßgeblich ist, daß sie im wesentlichen keine Katalase-Aktivität enthält.
Geeignete pflanzliche Herkunftsquellen sind beispielsweise Meerrettich, Sojabohnen, Feigen und Erdnüsse; geeignete Bakterienstämme zur Herstellung von Peroxidasen sind beispielsweise Acetobacter, Staphylococcen und Lactobacillen; auch aus Bäckerhefe gewonnene katalasefreie Peroxidasen sind geeignet.

Prinzipiell sind alle in der Klasse 1.11 der "Classification of the International Union of Biochemistry on the Nomenclature and Classification of Enzymes (1964)" aufgeführten Peroxidasen im Rahmen der vorliegenden Erfindung geeignet.
Besonders bevorzugt ist hierbei das unter der Nummer 1.11.1.7. klassifizierte Peroxidase-Enzym, jedoch können beispielsweise auch NAD-Peroxidase (1.11.1.1.), NADP-Peroxidase (1.11.1.2.), Fettsäure-Peroxidase (1.11.1.3.), Cytochrom-Peroxidase (1.11.1.5.) und Glutathion-Peroxidase (1.11.1.9.) verwendet werden.

Die bevorzugte Enzymaktivität bei der Anwendung beträgt von etwa 3 bis etwa 500 Peroxidase-Einheiten (U) pro 100 g der Enzymlösung. Der besonders bevorzugte Wert liegt bei etwa 10 bis etwa 150 U/100 g, insbesondere 15 bis 50 U/100 g Enzymzusammensetzung.

Berechnet auf reines Enzym, liegt die Aktivität bei mehr als 200 U/mg, insbesondere mehr als 250 U/mg (25°C).

Als "katalasefrei" im Rahmen der Erfindung gilt eine Peroxidase, deren Katalasegehalt bei weniger als 0,7 %, vorzugsweise unter 0,5 % der Gesamt-Peroxidase-Aktivität, beträgt.

Die Bestimmung der Peroxidase-Aktivität ist seit langem bekannt und in diversen Veröffentlichungen beschrieben.

Das erfindungsgemäße Verfahren zur Haarfärbung wird vorzugsweise so durchgeführt, daß die das Oxidationsfarbstoff-Vorprodukt enthaltende Zusammensetzung mit der Enzymzusammensetzung und der niedrig konzentrierten Oxidationsmittel-Zusammensetzung vermischt und die so erhaltene Farbmischung anschließend auf das Haar aufgebracht wird.

Alternativ dazu kann auch unmittelbar vor der Anwendung die Enzymzusammensetzung mit der Peroxidzusammensetzung vereinigt und diese mit dem Oxidationsfarbstoff-Vorprodukt gemischt werden.

Es ist auch möglich, das Enzym mit dem Oxidationsmittel oder dem Farbstoff-Vorprodukt in einer Zusammensetzung einzusetzen. Voraussetzung hierfür ist, daß eine der beiden Substanzen bis zur Einwirkung auf das Haar immobilisiert wird, was beispielsweise durch Einkapselung des Oxidationsmittels, insbesondere eines Peroxids, oder des Enzyms, geschieht, so daß dieses erst bei der Aufbringung auf das Haar, entweder durch mechanisches Aufbrechen der Kapsel oder chemisch (beispielsweise durch Variation des pH-Wertes) bedingte Freigabe freigesetzt wird.

Schließlich kann auch, in einem Mehrstufenverfahren, zunächst die Oxidationsfarbstoff-Vorprodukt-Zusammensetzung auf das Haar aufgebracht und anschließend mit der Enzym-/Oxidationsmittel-Zusammensetzung gleichzeitig oder nacheinander die Färbung auf dem Haar entwickelt werden.
Falls es sich bei dem Oxidationsmittel um ein bei Anwendung aufzulösendes Pulver oder eine Tablette handelt, kann das ebenfalls pulverförmige Peroxidase-Enzym auch diesem Pulver bzw. der Tablette zugesetzt werden.

Im Rahmen des erfindungsgemäßen Verfahrens können die an sich bekannten Haarfärbezusammensetzungen zum Einsatz gelangen, mit Ausnahme der Tatsache selbstverständlich, daß die Konzentration des Oxidationsmittels, insbesondere Wasserstoffperoxid, wesentlich herabgesetzt ist.

Die Konzentration des Peroxids liegt dabei zwischen etwa 0,01 und etwa 1, vorzugsweise 0,02 bis 0,5, insbesondere 0,02 bis etwa 0,1 Gew.-% der Oxidationsmittel-Zusammensetzung, berechnet als Wasserstoffperoxid.
Wird ein anderes Oxidationsmittel, beispielsweise ein Perborat, Harnstoffperoxid oder Melaminperoxid eingesetzt, muß die Konzentration entsprechend umgerechnet werden.

Aus der EP-A 310 675 sind bereits Haarpflegemittel-Zusammensetzungen bekannt, die als aktiven Bestandteil eine Oxidase der Gruppe Pyranose-Oxidase, Glucose-Oxidase, Glycerin-Oxidase, Milchsäure-Oxidase, Brenztraubensäure-Oxidase und Uricase enthalten, für die Sauerstoff einen Akzeptor darstellt.
Durch die Verwendung dieser Enzyme soll insbesondere der Einsatz von Peroxiden wie Wasserstoffperoxid in Fixiermitteln für Dauerwellen und Haarfärbemitteln auf Basis von Oxidationsfarbstoff-Vorprodukten überflüssig gemacht werden.
Die im Testbeispiel 1 sowie den Beispielen 1 - 10 der EP-A 310 675 verwendeten Färbezusammensetzungen enthalten demzufolge auch kein Wasserstoffperoxid, da diese Veröffentlichung, wie oben dargestellt, von einer anderen Aufgabenstellung ausgeht und deshalb mit der vorliegenden Erfindung in keinem näheren Zusammenhang steht.

Aus der DE-A 2 155 390 und den damit übereinstimmenden US-PSen 3 893 803 und 3 957 424 sind bereits Haarfärbeverfahren und -mittel bekannt, die dadurch gekennzeichnet sind, daß das Haar mit einer Lösung behandelt wird, die ein Peroxidase-Enzym, etwa 0,01 bis etwa 1,0 Gewichtsprozent Wasserstoffperoxid und ein Oxidationsfarbstoff-Vorprodukt enthält.

Diese Mittel und das damit durchzuführende Färbeverfahren haben sich bei der praktischen Anwendung nicht als verwendbar erwiesen, vermutlich deshalb, weil die Wichtigkeit der Abwesenheit von Katalase nicht erkannt wurde.
Im folgenden wird die Erfindung anhand von Beispielen näher illustriert:

### Beispiel 1

Strähnen aus Büffelhaar wurden mit einer Farbstoffvorprodukt-Zusammensetzung behandelt, die in einer üblichen Grundlage auf wäßriger Basis
0,25 Gew.-% p-Phenylendiamin und
0,30 Gew.-% Resorcin
enthielt.
Nach fünfminütiger Einwirkung dieser Lösung auf die Strähne wurde eine frisch hergestellte wäßrige Lösung aus
0,06 Gew.-% Wasserstoffperoxid und
16 U/100 mg Peroxidase (1.11.1.7),
eingestellt auf pH 7
im Verhältnis 1 : 1 zur Färbelösung auf das Haar aufgetragen. Nach weiterem 15-minütigem Einwirken und anschließendem Waschen wies das Haar eine glänzende dunkelbraune Färbung auf.
Wie Dehnungsmessungen des erfindungsgemäß behandelten Haares im Vergleich zu konventionell (mit 6%iger H₂O₂-Lösung) analog gefärbtem Haar ergaben, liegt die Elastizität des erfindungsgemäß behandelten Haares wesentlich höher, was ein klarer Nachweis für die verringerte Haarschädigung ist.

### Beispiel 2

Eine Farbstoffvorprodukt-Mischung aus
0,25 Gew.-% p-Phenylendiamin und
0,30 Gew.-% Resorcin,
in einer üblichen Gel-Grundlage, wurde auf Büffelhaar-Strähnen aufgetragen.

Anschließend wurden, nacheinander, gleiche Teile einer wäßrigen 0,06-prozentigen Wasserstoffperoxid-Lösung (gepuffert auf pH 7) und einer 0,3-prozentigen Agar-Agar-Lösung, enthaltend
16 U/100 g Peroxidase (1.11.1.7),
eingestellt auf pH 7 mit 0,1M KH₂PO₄/K₂HPO₄-Pufferlösung, aufgebracht.
Nach 15-minütiger Einwirkungszeit und Waschen wurde ein glänzender dunkelbrauner Farbton erreicht.
Dehnungsmessungen mit dem gefärbten Haar ergaben keinen Unterschied zu unbehandelten Haarsträhnen; eine Schädigung erfolgte demnach nicht.

### Beispiel 3

20 g einer Farbstoffvorprodukt-/Kuppler-Mischung, enthaltend
0,350 Gew.-% p-Toluylendiamine,
0,005 Gew.-% p-Aminophenol,
0,230 Gew.-% p-Amino-o-kresol
in einer üblichen wäßrigen Gel-Grundlage, wurden mit gleichen Teilen einer Lösung aus
0,06 Gew.-% Wasserstoffperoxid,
0,30 Gew.-% Agar-Agar
0,50 Gew.-% C₉-C₁₁-Alkylpolyglycosid (Kondensationsgrad x = 1,35)
@ 100,00 Gew.-% Wasser
bzw. 16 U/100 g Peroxidase (1.11.1.7)
0,30 Gew.-% Agar-Agar
0,50 Gew.-% C₉-C₁₁-Alkylpolyglycosid (Kondensationsgrad x = 1,35)
@ 100,00 Gew.-% Wasser,
vermischt und sofort auf menschliches Haar aufgebracht. Nach 15-minütigem Einwirken und Waschen wurde eine glänzende braun-violette Färbung ohne Haarschädigung erhalten.

Diese Versuche zeigen, daß die Färbedauer mit den erfindungsgemäßen Mitteln um etwa die Hälfte herabgesetzt werden kann.

Die Farbstoffvorprodukt-Zusammensetzungen sind dem Fachmann für die Erzielung der jeweils gewünschten Farbtöne geläufig. Es wird hierzu auch auf die einschlägigen Handbücher, beispielsweise die Monographie von K. Schrader, "Grundlagen und Rezepturen der Kosmetika", 2. Auflage (1989, Hüthig Buch Verlag), S. 782 - 815, verwiesen.

## Patentansprüche

1. Verfahren zur oxidativen Färbung von menschlichen Haaren, dadurch gekennzeichnet, daß die Färbung der Haare durch eine an sich bekannte Oxidationsfarbstoffvorprodukt-Zusarnrnensetzung zusammen mit einer 0,01 bis 1 Gew.-% Oxidationsmittel, berechnet als Wasserstoffperoxid, enthaltenden Entwickler-Zusammensetzung in Gegenwart einer katalasefreie Peroxidase enthaltendenden Zusammensetzung, deren Katalasegehalt weniger als 0,7% der Gesamt-Peroxidase-Aktivität beträgt, erfolgt.

2. Verfahren nach Anspruch 1, dadurch gekennzeichnet, daß zunächst eine OxidationsfarbstoffVorprodukt-Zusammensetzung auf das Haar aufgebracht und dieses anschließend gemeinsam oder nacheinander mit einer Oxidationsmittel-Zusammensetzung, die 0,01 bis 1 Gew.-%, vorzugsweise 0,02 bis 0,5 Gew.-%, eines Oxidationsmittels, berechnet als Wasserstoffperoxid, enthält, und einer katalasefreie Peroxidase enthaltenden Zusammensetzung behandelt wird.

3. Haarfärbemittel, bestehend aus einer Haarfarbstoff-Vorprodukt-Zusammensetzung und einem davon bis zur Anwendung räumlich getrennt gehaltenen Oxidationsmittel, das 0,01 bis 1 Gewichtsprozent eines Peroxids, berechnet auf Wasserstoffperoxid, enthält, dadurch gekennzeichnet, daß es in räumlich getrennter Zusammensetzung davon eine katalasefreie Peroxidase, deren Katalasegehalt weniger als 0,7% der Gesamt-Peroxidase-Aktivität beträgt, enthält.

4. Mittel nach Anspruch 3, gekennzeichnet durch einen Gehalt an 0,02 bis 0,2 Gewichtsprozent Wasserstoffperoxid, berechnet auf die Gesamtzusammensetzung.

5. Mittel nach Anspruch 3 oder 4, gekennzeichnet durch einen Gehalt an 3 bis 500 Peroxidase-Einheiten/100 g der Zusammensetzung.

6. Mittel nach einem der Ansprüche 3 bis 5, dadurch gekennzeichnet, daß es etwa 0,1 bis etwa 4,0 Gewichtsprozent mindestens eines nichtionischen Tensids enthält.

7. Mittel nach Anspruch 6, dadurch gekennzeichnet, daß es als nichtionisches Tensid ein Alkylglycosid der allgemeinen Formel
RO(OR¹)ₜZₓ,
worin R eine Alkylgruppe mit 8 bis 18 Kohlenstoffatomen, R¹ einen Ethylen- oder Propylenrest, z ein reduzierendes Saccharid mit 5 bis 6 Kohlenstoffatomen, t null bis 10 und x eins bis 5 bedeuten, enthält.

8. Mittel nach Anspruch 6, dadurch gekennzeichnet, daß es als nichtionisches Tensid ein Ethylenoxid/Propylenoxid-Copolykondensat enthält.

9. Mittel nach Anspruch 8, dadurch gekennzeichnet, daß es als Ethylenoxid/Propylenoxid-Copolykondensat ein solches der allgemeinen Formel 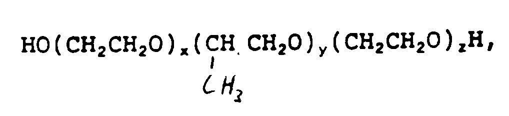 enthält, wobei die Durchschnittswerte für x und z jeweils zwischen 2 und 128 und für y zwischen 16 und 67 liegen.

10. Mittel nach Anspruch 9, dadurch gekennzeichnet, daß die Durchschnittswerte für x und z bei jeweils 98 und für y bei 67 liegen.

## Claims

1. Process for oxidative dyeing of human hair, characterized in that the dyeing process is effected by applying an oxidation dyestuff precursor composition known per se, in the presence of a catalase-free peroxidase, together with a developing substance containing an oxidizing compound of 0.01 to 1% by weight, calculated as hydrogen peroxide, to the hair.

2. Process according to claim 1, characterized in that first an oxidation dyestuff precursor composition is applied onto the hair, and then, either simultaneously or subsequently, the hair is treated with an oxidizing compound comprising 0.01 to 1% by weight, preferably 0.02 to 0.5% by wt. of an oxidant, calculated as hydrogen peroxide, and a catalase-free peroxidase.

3. Hair dye composition comprising a hair dyestuff precursor composition and an oxidizing compound kept separately until application, containing 0.01 to 1% by weight of a peroxide, calculated as hydrogen peroxide, characterized in that it comprises a composition of catalase-free peroxidase which is kept separate therefrom.

4. Composition according to claim 3, characterized in that it contains 0.02 to 0.1% by weight of hydrogen peroxide, calculated to the total composition.

5. Composition according to claim 3 or 4, characterized in that it contains 3 to 500 peroxidase units per 100g of the total composition.

6. Composition according to one of claims 3 to 5, characterized in that it comprises about 0.1 to about 4.0% by weight of at least one nonionic surfactant.

7. Composition according to claim 6, characterized in that it contains as a nonionic surfactant an alkyl glucoside of the general formula
RO(OR¹)ₜZₓ,
wherein R denotes an alkyl group with 8 to 18 carbon atoms, R¹ an ethylene or propylene residue, z a reducing saccharide with 5 to 6 carbon atoms, t is zero to ten and x is one to five.

8. Composition according to claim 6, characterized in that it contains an ethyleneoxide/propyleneoxide-copolycondensate as a nonionic surfactant.

9. Composition according to claim 8, characterized in that it contains as an ethyleneoxide/propyleneoxide-copolycondensate one of the general formula 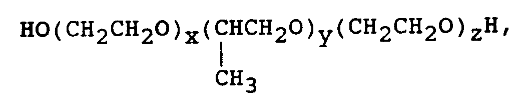 wherein the average values for x and z are between 2 and 128 and for y between 16 and 67.

10. Composition according to claim 9, characterized in that the average values for x and z are about 98 each, and for y about 67.

## Revendications

1. Procédé pour la coloration oxydante des cheveux humains, caractérisé en ce que la coloration des cheveux est effectuée à l'aide d'une composition de précurseur de colorant oxydant connue en elle-même conjointement avec une composition de développeur contenant de 0,01 à 1 % en poids d'agent oxydant, calculé en équivalent peroxyde d'hydrogène, en présence d'une composition contenant une peroxydase exempte de catalase, dont la teneur en catalase représente moins de 0,7 % de l'activité totale de peroxydase.

2. Procédé selon la revendication 1, caractérisé en ce qu'une composition de précurseur de colorant oxydant est d'abord appliquée sur les cheveux et que ceux-ci sont ensuite traités simultanément ou successivement par une composition d'agent oxydant qui contient de 0,01 à 1 % en poids, de préférence de 0,02 à 0,5 % en poids d'un agent oxydant, calculé en équivalent peroxyde d'hydrogène, et d'une composition contenant une peroxydase exempte de catalase.

3. Produit colorant pour cheveux, constitué par une composition de précurseur de colorant pour cheveux et d'un produit oxydant conservé séparément jusqu'à l'utilisation et contenant de 0,01 à 1% en poids d'un peroxyde, calculé en équivalent peroxyde d'hydrogène, caractérisé en ce qu'il contient dans la composition maintenue séparée, une peroxydase exempte de catalase, dont la teneur en catalase représente moins de 0,7% en poids de l'activité totale de la peroxydase.

4. Produit selon la revendication 3, caractérisé par une teneur en peroxyde d'hydrogène de 0,02 à 0,2 % en poids, par rapport à la composition totale.

5. Produit selon la revendication 3 ou 4, caractérisé par une teneur en unités peroxydase de 3 à 500 / 100 g de composition.

6. Produit selon l'une des revendications 3 à 5, caractérisé en ce qu'il contient d'environ 0,1 à environ 4,0 % en poids d'au moins un agent tensio-actif non ionique.

7. Produit selon la revendication 6, caractérisé en ce qu'il contient comme agent tensio-actif non ionique, un glycoside d'alkyle ayant la formule générale RO(OR¹)ₜZₓ, dans laquelle R représente un groupe alkyle ayant de 8 à 18 atomes de carbone, R¹ représente un radical éthylène ou propylène, Z représente un saccharide réducteur ayant de 5 à 6 atomes de carbone, t représente de 0 à 10 et x représente de 1 à 5.

8. Produit selon la revendication 6, caractérisé en ce qu'il contient comme agent tensioactif non ionique un copolycondensat d'oxyde d'éthylène et d'oxyde de propylène.

9. Produit selon la revendication 8, caractérisé en ce qu'il contient comme copolycondensat d'oxyde d'éthylène et d'oxyde de propylène, un composé ayant la formule 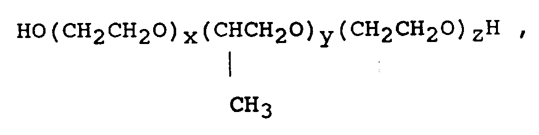 dans laquelle les valeurs moyennes sont respectivement entre 2 et 128 pour x et z et entre 16 et 67 pour y.

10. Produit selon la revendication 9, caractérisé en ce que les valeurs moyennes sont respectivement de 98 pour x et z et de 67 pour y.
